# EUROPEAN PATENT SPECIFICATION

(11) **EP 3 405 934 B1**
(45) Date of publication and mention of the grant of the patent: **30.12.2020**
(21) Application number: 17708034.8
(22) Date of filing: 14.02.2017
(51) Int. Cl.: H04W 4/021, G01N 33/00, G08B 21/14, H04W 4/80

(54) **USING BLUETOOTH BEACONS TO AUTOMATICALLY UPDATE THE LOCATION WITHIN A PORTABLE GAS DETECTOR'S LOGS**
VERWENDUNG VON BLUETOOTH-BAKEN ZUR AUTOMATISCHEN AKTUALISIERUNG DER POSITION INNERHALB DER PROTOKOLLE EINES TRAGBAREN GASDETEKTORS
UTILISATION DE BALISES BLUETOOTH POUR METTRE À JOUR AUTOMATIQUEMENT L'EMPLACEMENT DANS LES JOURNAUX D'UN DÉTECTEUR DE GAZ PORTABLE

(30) Priority: 25.02.2016 US 201615053812
(43) Date of publication of application: 28.11.2018
(60) Divisional application: 20201532.7
(73) Proprietor: Honeywell International Inc., Morris Plains, NJ 07950 (US)
(72) Inventor: JOHNSON, Kirk William, Morris Plains, New Jersey 07950 (US); JAVER, Mahdi, Morris Plains, New Jersey 07950 (US); MROSZCZAK, Stephen, Morris Plains, New Jersey 07950 (US)
(74) Representative: Haseltine Lake Kempner LLP
(86) International application number: PCT/US2017/017748
(87) International publication number: WO 2017/146934

(56) References cited:
- EP-A1- 3 131 008
- US-A1- 2011 037 599
- US-A1- 2011 043 373
- US-A1- 2011 161 044
- US-A1- 2013 045 759
- US-A1- 2014 349 707
- US-A1- 2015 237 419
- Anonymous ET AL: "LMP9100 - low power eletrochemical sensor", , 31 December 2014 (2014-12-31), XP055375228, Retrieved from the Internet: URL:http://www.ti.com/lit/ds/symlink/lmp91 000.pdf [retrieved on 2017-05-23]

## Description

### CROSS-REFERENCE TO RELATED APPLICATIONS

The present application is the National Stage of International Application No. PCT/US2017/017748 filed February 14, 2017 and entitled "Using Bluetooth Beacons To Automatically Update The Location Within A Portable Gas Detector's Logs," which claims priority to U.S. Patent Application No. 15/053,812 filed February 25, 2016, and entitled "Using Bluetooth Beacons To Automatically Update The Location Within A Portable Gas Detector's Logs,".

### STATEMENT REGARDING FEDERALLY SPONSORED RESEARCH OR DEVELOPMENT

Not applicable.

### REFERENCE TO A MICROFICHE APPENDIX

Not applicable.

### BACKGROUND

In hazardous work environments, workers may carry gas detectors with them as they work, to allow for detection of gas exposure. The gas detector may alert the user if exposure limits are reached while the user is wearing the gas detector. Gas detectors may comprise interfaces for communicating with the user, such as displays, lights, buzzers, and input buttons. Gas detectors may be configured with settings for alarms, exposure limits, display settings, light and buzzer settings, etc.

US20150237419A1 discloses a radiation exposure monitoring system comprising wireless dosimeter devices that are very energy efficient and that can communicate remotely with a remote host that in turn performs the dose calculations from the raw data transmitted by the wireless dosimeter devices. The system has a plurality of wireless dosimeter devices not equipped with a display screen, each comprising a integrated dosimeter, a control unit being in turn connected to a wireless transceiver for the transmission of data representative of the radiation detected by each dosimeter; at least one remote host comprising a wireless transceiver suitable for communicating with at least some of the transceivers of the dosimeter devices and with at least one remote host for tracking the wireless dosimeter.

US20110161044A1 discloses a method and apparatus for estimating the location of a portable device. The method includes the steps of providing a plurality of location anchor transceivers and a plurality of enhanced location anchor transceivers within a region, each of the plurality of location anchor transceivers and enhanced location anchor transceivers operating from respective predetermined locations, a portable transceiver within the region detecting and measuring a signal parameter of a signal from at least some of the plurality of location anchor transceivers and enhanced location anchor transceivers within the region, a location engine determining a location of the portable transceiver based upon the signal measurements of the portable transceiver and the respective predetermined locations of the plurality of location anchor transceivers and enhanced location anchor transceivers within the region, each of the plurality of enhanced location anchor transceivers detecting and measuring a signal parameter of a signal from at least some of the plurality of location anchor transceivers and other enhanced location anchor transceivers, the location engine receiving the measured signal parameters from each of the plurality of enhanced location anchor transceivers, the location engine calculating an estimated location for each of the plurality of enhanced location anchor transceivers from the measured signal parameters and the location engine calculating a location error for each of the plurality of enhanced location anchor transceivers based upon the estimated location and predetermined location of the enhanced location anchor transceiver.

US20110037599A1 discloses a system for providing real time locating and gas exposure monitoring. The system may include a memory, interface, and processor. The memory may store an alarm data item including an amount of gas exposure and a location identifier. The processor may receive the alarm data item from a sensor device of a first user. The processor may identify a relative location of the first user based on the location identifier. The processor may determine a second user located within a proximity of the first user. The processor may communicate to the second user the relative location of the first user and the gas exposure of the first user. The processor may receive, from the second user, an indication of whether an emergency responder should be contacted. The processor may initiate communication with an emergency responder if the indication indicates an emergency responder should be contacted.

### BRIEF DESCRIPTION OF THE DRAWINGS

For a more complete understanding of the present disclosure, reference is now made to the following brief description, taken in connection with the accompanying drawings and detailed description, wherein like reference numerals represent like parts.
FIG. 1 illustrates a gas detector device according to an embodiment of the disclosure;
FIG. 2 illustrates a facility map according to an embodiment of the disclosure; and
FIG. 3 illustrates a method according to an embodiment of the disclosure.

### DETAILED DESCRIPTION

It should be understood at the outset that although illustrative implementations of one or more embodiments are illustrated below, the disclosed systems and methods may be implemented using any number of techniques, whether currently known or not yet in existence. The disclosure should in no way be limited to the illustrative implementations, drawings, and techniques illustrated below, but may be modified within the scope of the appended claims along with their full scope of equivalents.

The following brief definition of terms shall apply throughout the application:
The term "comprising" means including but not limited to, and should be interpreted in the manner it is typically used in the patent context;
The phrases "in one embodiment," "according to one embodiment," and the like generally mean that the particular feature, structure, or characteristic following the phrase may be included in at least one embodiment of the present invention, and may be included in more than one embodiment of the present invention (importantly, such phrases do not necessarily refer to the same embodiment);
If the specification describes something as "exemplary" or an "example," it should be understood that refers to a non-exclusive example;
The terms "about" or "approximately" or the like, when used with a number, may mean that specific number, or alternatively, a range in proximity to the specific number, as understood by persons of skill in the art field; and
If the specification states a component or feature "may," "can," "could," "should," "would," "preferably," "possibly," "typically," "optionally," "for example," "often," or "might" (or other such language) be included or have a characteristic, that particular component or feature is not required to be included or to have the characteristic. Such component or feature may be optionally included in some embodiments, or it may be excluded.

Embodiments of the disclosure include systems and methods for updating the location information for a gas detector device. A gas detector device may comprise a wireless receiver operable to receive information from one or more wireless beacons. In some cases, the wireless beacons may comprise location information.

Workers who use gas detectors are sometimes required to indicate their location to the gas detector. For example, the worker may manually enter their current location into the detector. Some detectors may allow a worker to change the location through a series of menus and button presses. Other devices may only have one button and require the user to connect to a computer to change the location. This can be incredibly time consuming for the user.

To simplify the process of updating the location of a gas detector, wireless beacons (such as low power Bluetooth) may be installed at key locations within a facility. These beacons may advertise packets of information with information regarding the location of the beacon, and therefore the user. The user's gas detector may receive these advertisements and automatically update the location field to match the beacon advertisement.

The wireless beacon may be designed to have a very long run time (for example, 2 or more years), and would continuously advertise its location. A gas detector may be designed with a wireless (or BLE) reader that looks for these advertisements, receives the advertisements, and changes its location based on advertisements it sees.

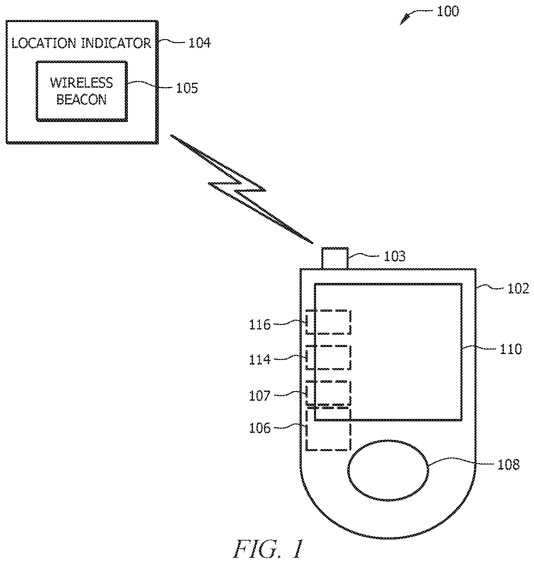

Referring now to FIG. 1, a system 100 is shown, including a gas detector device 102, where the gas detector device 102 comprises a wireless receiver 106. In some embodiments, the gas detector device 102 may comprise one or more sensors 103, a user interface 110, and one or more buttons 108. The gas detector device 102 may be operable to detect, via the sensors 103, any harmful gases or chemicals in the air near a user who is carrying the gas detector device 102. For example, the sensors 103 can detect various types of information such as chemical components of an environment, environmental conditions (e.g., temperature, pressure, wind speed, wind direction, etc.), vibration levels, noise levels, biometric parameters (e.g., heart rate, body temperature, respiration rate, etc.), location (e.g., including 2-dimensional and/or 3-dimensional position), and the like.

Additionally, the sensors 103 may include, but are not limited to, radiation detectors, smoke detectors, and detectors for determining abnormally low oxygen content in the atmosphere, as well as a wide variety of detectors for detecting chemically hazardous or flammable gases such as, for example, hydrogen sulfide, ammonia, carbon monoxide, natural gas, phosgene, organic compounds (e.g., volatile organic compounds, etc.), and so forth. The gas sensors 103 can also be configured to include integrated wireless communications and the ability to periodically and under event conditions, report the location information, time information, and gas concentration level information wirelessly. In some embodiments, the gas detector device 102 may be operable to alert a user based on the input from the one or more sensors 103. In some embodiments, a user may control the gas detector device 102 and input information via the user interface 110 and/or button 108. Additionally, the user may receive information via the user interface 110.

The gas detector device 102 may also comprise a processor 114 and a memory 116. The processor 114 may be operable to receive information from the one or more sensors 103. The processor 114 may also be operable to receive information from the wireless receiver 106. In some embodiments, the gas detector device 102 may also comprise a wireless transmitter 107 operable to communicate wirelessly. In some embodiments, the gas detector device 102 may communicate with a remote monitoring station or other remote device. The portable gas detector device 102 may communicate wirelessly, over a wireless fidelity (Wi-Fi) network, via Bluetooth, Near Field Communication (NFC) or another wireless connection.

The system 100 may also comprise one or more location indicators 104, where the location indicators 104 may be placed at critical or important locations, such as at the entrances and/or exits of specific locations. The location indicator 104 may comprise a wireless beacon 105 operable to broadcast information about the location indicator 104. In some embodiments, the wireless beacon 105 may comprise a Bluetooth (or BLE) wireless beacon. The wireless beacon 105 may be received by the wireless receiver 106 of the gas detector device 102. The wireless receiver 106 may forward the received wireless beacon 105 to the processor 114, where the processor 114 may store the location information from the wireless beacon 105 in the memory 116, and where the processor 114 may associate new information received from the sensors 103 with the updated location information. In some embodiments, the wireless beacon 105 may communicate a location ID, where the gas detector may access a table with the location ID to input information associated with that location ID. In some embodiments, the wireless beacon 105 may communicate a location ID, where the gas detector may simply store the location ID in the memory.

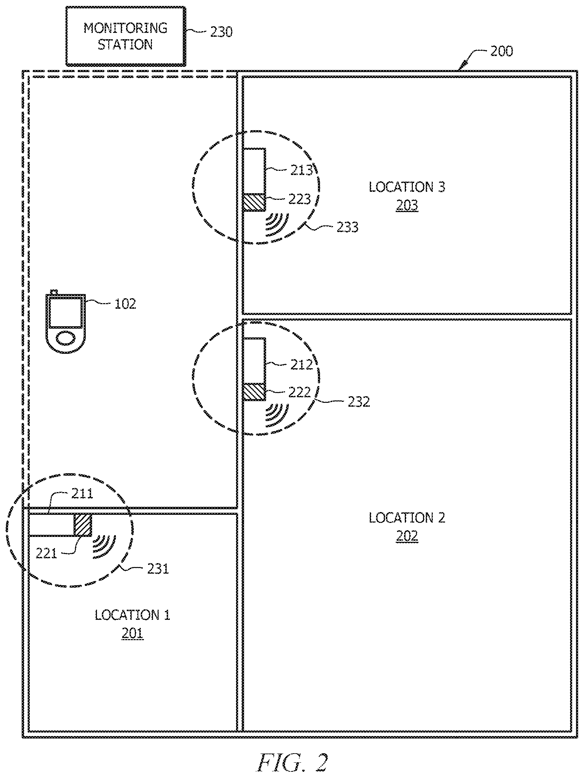

Referring to FIG. 2, a facility 200 is shown comprising multiple locations or zones. The locations may comprise one or more critical areas, such as entrances or exits. For example, a first location 201 may comprise an entrance 211, a second location 202 may comprise an entrance 212, and a third location 203 may comprise an entrance 213. In some embodiments, the facility 200 may comprise location indicators 221, 222, and 223 located at or near one or more of the entrances 211, 212, 213. The location indicators 221, 222, and 223 may be similar to the location indicator 104 described in FIG. 1, where the location indicators may comprise wireless beacons operable to broadcast information about the location(s).

In some embodiments, the gas detector device 102 may travel, with a user, between the different locations. When the user enters a new location 201, the gas detector device 102 may receive the wireless beacon from the location indicator 221 at the entrance 211 of the location 201. Then, the gas detector device 102 may update the location information stored by the gas detector device 102, where all subsequent readings taken by the (sensors of the) gas detector device 102 may be associated with the updated location information. Similarly, if the gas detector device 102 travels with the user to other locations 202 or 203, the gas detector device 102 may receive new location information from the location indicators 222 and 223, and would update the location information stored on the gas detector device 102 accordingly.

In some embodiments, the gas detector device 102 may indicate to a user that a location update is requested or received, and a user may be required to input a confirmation for the update to proceed. In some embodiments, the location indicators 221, 222, 223 may comprise a physical sign or notification for the user that they are entering a new location and also receiving a wireless beacon to their gas detector device 102.

In some embodiments, the location indicators 221, 222, 223 may have specific ranges 231, 232, 233 that encompass the specific area that is included in the location information in the wireless beacon of the location indicator. For example, the range 23 1 of the location indicator 221 may be adjusted to cover only the entrance 211 of the location 201, so as not to interfere with other location indicators, or incorrectly indicate the location to a device that is not entering the location 201.

In some embodiments, the gas detector device 102 may also communicate the received location information to a remote monitoring station 230. The remote monitoring station 230 may be monitored by a supervisor, for example. In other embodiments, the location information may be stored locally on the gas detector device 102 and may be accessed later by a monitor or monitoring station.

In some embodiments, a safety communicator (e.g., a communication device) comprising a data collection and communication application may be used to collect the sensor data and communicate the sensor data to the various elements of the system. In some embodiments, the data from the gas detector device 102 can be relayed through a communication module to a server, where the data can be combined to provide an overall view of a worker's risk factor. Various information such as alarms, notifications, information (e.g., maintenance protocols, instructions, live help, etc.), and the like can be relayed back to the worker through the system.

The safety communicator may provide a data connection to a data analytics server and/or a database through a network. The safety communicator may be wirelessly coupled to the network through an access point such as a Wi-Fi, Bluetooth, or cellular connection (e.g., through a wireless service tower). In some embodiments, the network may be the Internet representing a worldwide collection of networks and gateways that use the Transmission Control Protocol/Internet Protocol (TCP/IP) suite of protocols to communicate with one another. In some embodiments, the system 100 may also be implemented as a number of different types of networks such as, for example, an intranet, a local area network (LAN), or a wide area network (WAN).

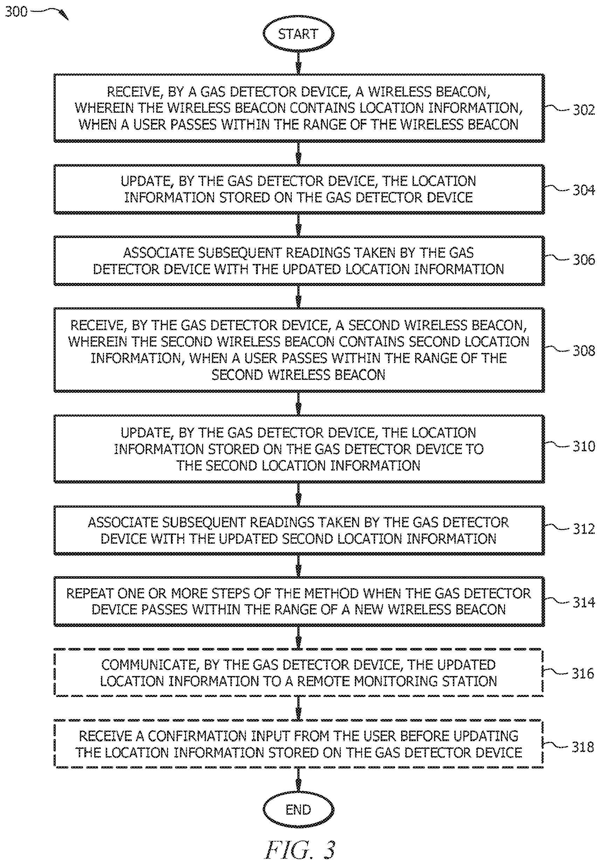

FIG. 3 illustrates a method for updating the location information on a gas detector device. In some embodiments, one or more steps of the method 300 may occur in different orders than shown in FIG. 3. At step 302, the gas detector device 102 may receive a wireless beacon when the gas detector device passes within the range of the wireless beacon, where the wireless beacon contains location information. At step 304, the gas detector device may update the location information stored on the gas detector device using the location information from the wireless beacon. At step 306, the gas detector device may associate subsequent readings taken by one or more sensors of the gas detector device with the updated location information.

In some embodiments, the gas detector device may receive more than one wireless beacon, where the wireless beacons may be received at different areas within a facility. At step 308, the gas detector device may receive a second wireless beacon, where the second wireless beacon contains second location information, when the gas detector device passes within the range of the second wireless beacon. At step 310, the gas detector device may update the location information stored on the gas detector device using the second location information from the second wireless beacon. At step 312, the gas detector device may associate subsequent readings taken by one or more sensors of the gas detector device with the updated second location information.

In some embodiments, at step 314, one or more steps of the method may be repeated when the gas detector device passes within the range of a new wireless beacon. Optionally, at step 316, the gas detector device may communicate the updated location information to a remote monitoring station. In some embodiments, step 316 may occur after step 304 and/or after step 310. Optionally, at step 318, the gas detector device may receive a confirmation input from the user before updating the location information stored on the gas detector device. In some embodiments, step 318 may occur before step 304 and/or before step 310.

In some embodiments, the wireless beacon may be located at one or more critical areas of a facility. In some embodiments, the wireless beacon may be located at one or more entrances to locations within a facility. In some embodiments, the gas detector device may receive the wireless beacon via a wireless receiver incorporated into the gas detector device. In some embodiments, updating the location information stored on the gas detector device occurs automatically.

Some embodiments of the disclosure may comprise a gas detector device comprising one or more sensors operable to detect gases in the air around the gas detector device; a wireless receiver operable to receive one or more wireless beacons; a memory; and a processor operable to receive and store readings from the one or more sensors of the gas detector device; receive location information from the wireless beacons; update the location information stored in the memory of the gas detector device using the received location information; and associate subsequent readings from the one or more sensors with the updated location information.

In some embodiments, the processor may be further operable to receive second location information from a second wireless beacon; update the location information stored in the memory of the gas detector device using the received second location information; and associate subsequent readings from the one or more sensors with the updated second location information. In some embodiments, the one or more wireless beacons are located at critical areas of a facility. In some embodiments, the one or more wireless beacons are located at one or more entrances to locations within a facility. In some embodiments, the one or more wireless beacons are incorporated into location indicators. In some embodiments, the location indicators comprise a sign or notification for the user that they are entering a new location, and therefore receiving a wireless beacon to their gas detector device. In some embodiments, the gas detector device may further comprise a wireless transmitter, where the processor is further operable to communicate the updated location information to a remote monitoring station. In some embodiments, updating the location information occurs automatically. In some embodiments, the processor is further operable to present a confirmation message to the user (via a user interface of the gas detector device); and receive a confirmation input from the user before updating the location information stored on the gas detector device.

Some embodiments of the disclosure may comprise a method for updating the location information on a gas detector device, the method comprising receiving, by a wireless receiver of a gas detector device, a wireless beacon, when the gas detector device passes within the range of the wireless beacon, where the wireless beacon contains location information; updating, by a processor of the gas detector device, the location information stored on the gas detector device using the location information from the wireless beacon; and associating, by the processor of the gas detector device, subsequent readings taken by one or more sensors of the gas detector device with the updated location information.

In some embodiments, the method may further comprise receiving, by the wireless receiver of the gas detector device, a second wireless beacon, when the gas detector device passes within the range of the second wireless beacon, where the second wireless beacon contains second location information; updating, by the processor of the gas detector device, the location information stored on the gas detector device using the second location information from the second wireless beacon; and associating, by the processor of the gas detector device, subsequent readings taken by the one or more sensors of the gas detector device with the updated second location information.

The scope of protection underlying the present invention is delimited by the appended claims.

## Claims

1. A method for updating location information on a gas detector device (102), the method comprising:
receiving, by a gas detector device (102), a wireless beacon (105), when the gas detector device (102) passes within the range of the wireless beacon (105), wherein the wireless beacon (105) contains location information;
presenting, by the gas detector device (102), a confirmation message for updating the location information, to a user via a user interface (110) of the gas detector device (102);
updating, by the gas detector device (102), the location information stored on the gas detector device (102) using the location information from the wireless beacon (105) when the user provides a confirmation input in response to the confirmation message;
associating, by the gas detector device (102), subsequent readings taken by one or more sensors (103) of the gas detector device (102) with the updated location information, wherein the readings are stored within the gas detector device (102);
receiving, by the gas detector device (102), a second wireless beacon (105), when the gas detector device (102) passes within the range of the second wireless beacon (105), wherein the second wireless beacon (105) contains second location information;
presenting, by the gas detector device (102), a second confirmation message for updating the location information, to a user via a user interface (110) of the gas detector device (102);
updating, by the gas detector device (102), the location information stored on the gas detector device (102) using the second location information from the second wireless beacon (105) when the user provides a second confirmation input in response to the second confirmation message; and
associating subsequent readings taken by one or more sensors (103) of the gas detector device (102) with the updated second location information, wherein the readings are stored within the gas detector device (102).

2. The method of claim 1 further comprising repeating one or more steps of the method when the gas detector device (102) passes within a range of a new wireless beacon (105).

3. The method of claim 1 further comprising communicating, by the gas detector device (102), the updated location information to a remote monitoring station (230).

4. The method of claim 1, wherein the wireless beacon (105) is located at one or more critical areas of a facility (200).

5. The method of claim 1, wherein the wireless beacon (105) is located at one or more entrances (211, 212, 213) to locations within a facility (200).

6. The method of claim 1, wherein the gas detector receives the wireless beacon (105) via a wireless receiver (106) incorporated into the gas detector device (102).

7. A gas detector device (102) comprising:
one or more sensors (103) operable to detect gases in the air around the gas detector device (102);
a wireless receiver (106) operable to receive one or more wireless beacons (105);
a memory (116); and
a processor (114) operable to:
receive and store readings from the one or more sensors (103) of the gas detector (102);
receive location information from the wireless beacons (105);
present a confirmation message to the user via a user interface (110) of the gas detector device (102);
receive a confirmation input from the user before updating the location information stored on the gas detector device (102);
update the location information stored in the memory (116) of the gas detector device (102) using the received location information;
associate subsequent readings from the one or more sensors (103) with the updated location information;
receive second location information from a second wireless beacon (105);
present a second confirmation message to the user via a user interface (110) of the gas detector device (102);
receive a second confirmation input from the user before updating the location information stored on the gas detector device (102);
update the location information stored in the memory (116) of the gas detector device (102) using the received second location information; and
associate subsequent readings from the one or more sensors (103) with the updated second location information.

8. The gas detector device (102) of claim 7 wherein the one or more wireless beacons (105) are located at critical areas of a facility (200).

9. The gas detector device (102) of claim 7 wherein the one or more wireless beacons (105) are located at one or more entrances (211, 212, 213) to locations within a facility (200).

10. The gas detector device (102) of claim 7, wherein the one or more wireless beacons (105) are incorporated into location indicators (104).

## Patentansprüche

1. Verfahren zum Aktualisieren von Positionsinformationen auf einer Gasdetektorvorrichtung (102), wobei das Verfahren umfasst:
Empfangen durch eine Gasdetektorvorrichtung (102) einer Funkbake (105), wenn die Gasdetektorvorrichtung (102) innerhalb der Reichweite der Funkbake (105) durchgeht, wobei die Funkbake (105) Positionsinformationen enthält;
Präsentieren durch die Gasdetektorvorrichtung (102) einer Bestätigungsnachricht zum Aktualisieren der Positionsinformationen bei einem Benutzer über eine Benutzeroberfläche (110) der Gasdetektorvorrichtung (102);
Aktualisieren durch die Gasdetektorvorrichtung (102) der Positionsinformationen, die auf der Gasdetektorvorrichtung (102) gespeichert sind, unter Verwenden der Positionsinformationen von der Funkbake (105), wenn der Benutzer eine Bestätigungseingabe als Reaktion auf die Bestätigungsnachricht bereitstellt;
Assoziieren durch die Gasdetektorvorrichtung (102) nachfolgender Ablesungen, die von einem oder mehreren Sensoren (103) der Gasdetektorvorrichtung (102) erhoben werden, zu den aktualisierten Positionsinformationen, wobei die Ablesungen innerhalb der Gasdetektorvorrichtung (102) gespeichert werden;
Empfangen durch die Gasdetektorvorrichtung (102) einer zweiten Funkbake (105), wenn die Gasdetektorvorrichtung (102) innerhalb der Reichweite der zweiten Funkbake (105) durchgeht, wobei die zweite Funkbake (105) zweite Positionsinformationen enthält;
Präsentieren durch die Gasdetektorvorrichtung (102) einer zweiten Bestätigungsnachricht zum Aktualisieren der Positionsinformationen bei einem Benutzer über eine Benutzeroberfläche (110) der Gasdetektorvorrichtung (102);
Aktualisieren durch die Gasdetektorvorrichtung (102) der Positionsinformationen, die auf der Gasdetektorvorrichtung (102) gespeichert sind, unter Verwenden der zweiten Positionsinformationen von der zweiten Funkbake (105), wenn der Benutzer eine zweite Bestätigungseingabe als Reaktion auf die zweite Bestätigungsnachricht bereitstellt; und
Assoziieren nachfolgender Ablesungen, die von einem oder mehreren Sensoren (103) der Gasdetektorvorrichtung (102) erhoben werden, mit den aktualisierten zweiten Positionsinformationen, wobei die Ablesungen innerhalb der Gasdetektorvorrichtung (102) gespeichert werden.

2. Verfahren nach Anspruch 1, das ferner das Wiederholen eines oder mehrerer Schritte des Verfahrens umfasst, wenn die Gasdetektorvorrichtung (102) innerhalb einer Reichweite einer neuen Funkbake (105) durchgeht.

3. Verfahren nach Anspruch 1, das ferner das Übermitteln durch die Gasdetektorvorrichtung (102) der aktualisierten Positionsinformationen an eine Fernüberwachungsstation (230) umfasst.

4. Verfahren nach Anspruch 1, wobei die Funkbake (105) in einem oder mehreren kritischen Bereichen einer Einrichtung (200) positioniert ist.

5. Verfahren nach Anspruch 1, wobei die Funkbake (105) an einem oder mehreren Eingängen (211, 212, 213) zu Positionen innerhalb einer Einrichtung (200) positioniert ist.

6. Verfahren nach Anspruch 1, wobei der Gasdetektor die Funkbake (105) über einen Funkempfänger (106) empfängt, der in die Gasdetektorvorrichtung (102) eingebaut ist.

7. Gasdetektorvorrichtung (102), umfassend:
einen oder mehrere Sensoren (103), die betreibbar sind, um Gase in der Luft um die Gasdetektorvorrichtung (102) zu erfassen;
einen Funkempfänger (106), der betreibbar ist, um eine oder mehrere Funkbaken (105) zu empfangen;
einen Speicher (116); und
einen Prozessor (114), der betreibbar ist, um:
Ablesungen von dem einen oder den mehreren Sensoren (103) des Gasdetektors (102) zu empfangen und zu speichern;
Positionsinformationen von den Funkbaken (105) zu empfangen;
eine Bestätigungsnachricht an den Benutzer über eine Benutzeroberfläche (110) der Gasdetektorvorrichtung (102) zu präsentieren;
eine Bestätigungseingabe von dem Benutzer zu empfangen, bevor die Positionsinformationen, die auf der Gasdetektorvorrichtung (102) gespeichert sind aktualisiert werden;
die Positionsinformationen, die in dem Speicher (116) der Gasdetektorvorrichtung (102) gespeichert sind, unter Verwenden der empfangenen Positionsinformationen zu aktualisieren;
nachfolgende Ablesungen von dem einen oder den mehreren Sensoren (103) mit den aktualisierten Positionsinformationen zu assoziieren;
zweite Positionsinformationen von einer zweiten Funkbake (105) zu empfangen;
dem Benutzer eine zweite Bestätigungsnachricht über eine Benutzeroberfläche (110) der Gasdetektorvorrichtung (102) zu präsentieren;
eine zweite Bestätigungseingabe von dem Benutzer zu empfangen, bevor die Positionsinformationen, die auf der Gasdetektorvorrichtung (102) gespeichert sind, aktualisiert werden;
die Positionsinformationen, die in dem Speicher (116) der Gasdetektorvorrichtung (102) gespeichert sind, unter Verwenden der empfangenen zweiten Positionsinformationen zu aktualisieren; und
nachfolgende Ablesungen von dem einen oder den mehreren Sensoren (103) mit den aktualisierten zweiten Positionsinformationen zu assoziieren.

8. Gasdetektorvorrichtung (102) nach Anspruch 7, wobei die eine oder die mehreren Funkbaken (105) in kritischen Bereichen einer Einrichtung (200) positioniert ist/sind.

9. Gasdetektorvorrichtung (102) nach Anspruch 7, wobei die eine oder die mehreren Funkbaken (105) an einem oder mehreren Eingängen (211, 212, 213) zu Positionen innerhalb einer Einrichtung (200) positioniert ist/sind.

10. Gasdetektorvorrichtung (102) nach Anspruch 7, wobei die eine oder die mehreren Funkbaken (105) in Positionsanzeiger (104) eingebaut ist/sind.

## Revendications

1. Procédé de mise à jour d'informations de localisation sur un dispositif détecteur de gaz (102), le procédé comprenant :
la réception, par un dispositif détecteur de gaz (102), d'une balise sans fil (105), lorsque le dispositif détecteur de gaz (102) passe à portée de la balise sans fil (105), la balise sans fil (105) contenant des informations de localisation ;
la présentation, par le dispositif détecteur de gaz (102), d'un message de confirmation permettant de mettre à jour les informations de localisation, à un utilisateur par le biais d'une interface utilisateur (110) du dispositif détecteur de gaz (102) ;
la mise à jour, par le dispositif détecteur de gaz (102), des informations de localisation stockées sur le dispositif détecteur de gaz (102) à l'aide des informations de localisation de la balise sans fil (105) lorsque l'utilisateur fournit une entrée de confirmation en réponse au message de confirmation ;
l'association, par le dispositif détecteur de gaz (102), des lectures ultérieures prises par un ou par plusieurs capteurs (103) du dispositif détecteur de gaz (102) aux informations de localisation mises à jour, les lectures étant stockées à l'intérieur du dispositif détecteur de gaz (102) ;
la réception, par le dispositif détecteur de gaz (102), d'une seconde balise sans fil (105), lorsque le dispositif détecteur de gaz (102) passe à portée de la seconde balise sans fil (105), la seconde balise sans fil (105) contenant une seconde information de lieu ;
la présentation, par le dispositif détecteur de gaz (102), d'un second message de confirmation, permettant de mettre à jour les informations de localisation, à un utilisateur par le biais d'une interface utilisateur (110) du dispositif détecteur de gaz (102) ;
la mise à jour, par le dispositif détecteur de gaz (102), des informations de localisation stockées sur le dispositif détecteur de gaz (102) à l'aide des secondes informations de localisation de la seconde balise sans fil (105) lorsque l'utilisateur fournit une seconde entrée de confirmation en réponse à la seconde confirmation message ; et
l'association de lectures ultérieures prises par un ou par plusieurs capteurs (103) du dispositif détecteur de gaz (102) avec les secondes informations d'emplacement mises à jour, les lectures étant stockées à l'intérieur du dispositif détecteur de gaz (102).

2. Procédé selon la revendication 1, comprenant en outre la répétition d'une ou de plusieurs étapes du procédé lorsque le dispositif détecteur de gaz (102) passe à l'intérieur d'une plage d'une nouvelle balise sans fil (105).

3. Procédé selon la revendication 1, comprenant en outre la communication, par le dispositif détecteur de gaz (102), des informations de localisation mises à jour à une station de surveillance à distance (230).

4. Procédé selon la revendication 1, dans lequel la balise sans fil (105) est située au niveau d'une ou de plusieurs zones critiques d'une installation (200).

5. Procédé selon la revendication 1, dans lequel la balise sans fil (105) est située à une ou à plusieurs entrées (211, 212, 213) vers des emplacements situés à l'intérieur d'une installation (200).

6. Procédé selon la revendication 1, dans lequel le détecteur de gaz reçoit la balise sans fil (105) par le biais d'un récepteur sans fil (106) incorporé dans le dispositif détecteur de gaz (102).

7. Dispositif détecteur de gaz (102) comprenant :
un ou plusieurs capteurs (103) utilisable pour détecter des gaz dans l'air autour du dispositif détecteur de gaz (102) ;
un récepteur sans fil (106) utilisable pour recevoir une ou plusieurs balises sans fil (105) ;
une mémoire (116) ; et
un processeur (114) utilisable :
pour recevoir et pour stocker des lectures provenant du ou des capteurs (103) du détecteur de gaz (102) ;
pour recevoir des informations de localisation des balises sans fil (105) ;
pour présenter un message de confirmation à l'utilisateur par le biais d'une interface utilisateur (110) du dispositif détecteur de gaz (102) ;
pour recevoir une entrée de confirmation de l'utilisateur avant de mettre à jour les informations de localisation stockées sur le dispositif détecteur de gaz (102) ;
pour mettre à jour les informations de localisation stockées dans la mémoire (116) du dispositif détecteur de gaz (102) à l'aide des informations de localisation reçues ;
pour associer les lectures suivantes du ou des capteurs (103) aux informations mises à jour de localisation ;
pour recevoir des secondes informations d'emplacement d'une seconde balise sans fil (105) ;
pour présenter un second message de confirmation à l'utilisateur par le biais d'une interface utilisateur (110) du dispositif détecteur de gaz (102) ;
pour recevoir une seconde entrée de confirmation de l'utilisateur avant de mettre à jour les informations de localisation stockées sur le dispositif détecteur de gaz (102) ;
pour mettre à jour les informations de localisation stockées dans la mémoire (116) du dispositif détecteur de gaz (102) à l'aide des secondes informations de localisation reçues ; et
pour associer des lectures ultérieures du ou des capteurs (103) aux secondes informations mises à jour de localisation.

8. Dispositif détecteur de gaz (102) selon la revendication 7, dans lequel la ou les balises sans fil (105) sont situées au niveau de zones critiques d'une installation (200).

9. Dispositif détecteur de gaz (102) selon la revendication 7, dans lequel la ou les balises sans fil (105) sont situées à une ou à plusieurs entrées (211, 212, 213) vers des emplacements à l'intérieur d'une installation (200).

10. Dispositif détecteur de gaz (102) selon la revendication 7, dans lequel la ou les balises sans fil (105) sont incorporées dans des indicateurs d'emplacement (104).
